# NEW EUROPEAN PATENT SPECIFICATION

(11) **EP 0 092 876 B2**
(45) Date of publication and mention of the opposition decision: **03.02.1999**
(45) Mention of the grant of the patent: 01.10.1986
(21) Application number: 83200573.0
(22) Date of filing: 20.04.1983
(51) Int. Cl.: C07H 15/04, B01D 1/22

(54) **Process of purifying alkylpolysaccharides**
Verfahren zur Reinigung von Alkylpolysacchariden
Procédé de purification d'alkylpolysaccharides

(30) Priority: 26.04.1982 US 371693
(43) Date of publication of application: 02.11.1983
(73) Proprietor: THE PROCTER & GAMBLE COMPANY, Cincinnati Ohio 45202 (US)
(72) Inventor: Mao, Mark Hsiang K., Cincinnati, OH 45241 (US); Weeman, John Michael, Cincinnati, OH 45211 (US); Miller, Larry Eugene, Maderia, Ohio 45243 (US)
(74) Representative: Lawrence, Peter Robin Broughton

(56) References cited:
- EP-A- 77 167
- DE-A- 1 905 523
- DE-A- 3 001 064
- US-A- 4 053 006
- US-A- 4 223 129
- Chemical Engineering vol 13, 1965, pp. 175-190
- Kirk-Othmer Encyclopedia of Chemical Technology 2nd Ed., 1966, vol. 9, p. 459 and 3rd Ed., 1980, vol. 12, p. 135

## Description

### Technical Field

This invention relates to an improved process for making alkylpolysaccharides in which the alkyl group contains from 12 to 18 carbon atoms and the polysaccharide chain contains from 1 ½ to 3 saccharide units on the average. The improved process gives a good polysaccharide chain length distribution and a good color. The process can be used to prepare highly efficient alkylpolysaccharides useful as detergent surfactants and foam builders for anionic detergent surfactants.

Methyl glycosides are made in the examples of US-A-4,223,129. It is stated that if desired the products can be isolated by thin film evaporation.

Thin film evaporation is described in, for example, Chemical Engineering 13 (1965) pages 175-190 and Brochure EV24 "Luwa Thin Film Evaporator" 1970. A wiped film evaporator is disclosed in, e.g., US-A-4,053,066. Kirk-Othmer, Encyclopedia of Chemical Technology second edition 1966 volume 9 page 459 and third edition 1980 volume 12 page 135 discuss the Reynolds numbers associated with turbulence.

In DE-A-1,905,523, a process for removing fatty alcohol from a mixture with alkyl polysaccharide by heating under vacuum is disclosed.

Distillation of fatty alcohols from certain fatty alkyl polyglycosides is described in US-A-3,547,828.

In DE-A-3,001,064 the problem associated with removing fatty alcohol from an alkyl polysaccharide without causing an increase in colour is described.

EP-A-0077167 (not published at the priority date hereof) describes a particular catalytic process for the preparation of surface active glycosides and mentions that the "elimination of excess alcohol can be accomplished by one of the known methods such as vacuum distillation, molecular distillation and thin film evaporation".

### Summary of the Invention

Claim 1 refers to a process of making purified alkyl polysaccharide by removing alcohol from a mixture with an alkyl polysaccharide by heating the mixture under vacuum in a thin film evaporator wherein the saccharide is glucoside or fructoside, characterised in that the alkyl group contains 12 to 18 carbons and the alcohol is the corresponding fatty alcohol, the purified alkyl polysaccharide has an average chain length of 1½ to 3 and includes less than 10% of C₁₋₅ alkyl saccharide and polysaccharide and less than 10% of alkyl polysaccharide having a chain length of 6 or more and has an alkyl monosaccharide content of less than 60% and a fatty alcohol content of less than 2%, the thin film evaporator provides, in operation, a Reynolds number of at least 20,000 and a film thickness of less than 10mm, and a temperature of from 160°C to 200°C and a vacuum of from 0.1 to 20mm of mercury (13.3 to 2660 Pa), and the mixture is neutralised before the removal of fatty alcohol.

The present invention relates to a process for preparing a long chain alkyl polysaccharide comprising the essential step of reacting a short chain alkylsaccharide of a reducing saccharide containing from five to six carbon atoms in which the alkyl contains from one to five carbon atoms with a long chain fatty alcohol containing from 12 to 18 carbon atoms at a reaction temperature of from 90°C to 120°C in the presence of an acid catalyst, the resulting short chain alcohol being removed, preferably under a vacuum, and preferably as rapidly as possible, with the catalyst being destroyed by adding an alkaline material after at least 90% of the short chain alkyl saccharide has been destroyed, the extent of destruction can be determined by measuring the resulting short chain alcohol which has been removed, or, preferably, by measuring the percent of water in the distillate, and before the average polysaccharide chain length exceeds 3.

In particular, the invention relates to a process of removing unreacted long chain fatty alcohol in the product from the above step by heating the mixture under vacuum in a thin film evaporator providing, in operation, a Reynolds number of at least 20,000 and a film thickness of less than 10 mm, and a temperature of from 160°C to 200°C and a vacuum of from 0.1 to 20 mm of mercury. (13.3 to 2660 Pa). The unreacted fatty alcohol is reduced to a level of less than 2%, preferably less than 1/2%.

The combination of the above two steps can be used to provide a superior alkylpolysaccharide for use as a detergent surfactant in which the alkyl group contains from 12 to 18, preferably from 12 to 14 carbon atoms, the average polysaccharide chain length is from 1½ to 3, preferably from 1.6 to 2-3/4 saccharide units, the level of short chain alkylsaccharide and polysaccharide is less than 10%, the amount of alkylpolysaccharide in which the saccharide chain length is 6 or greater is less than 10%, preferably less than 5%, the alkylmonosaccharide content is less than 60%, preferably less than 50%, and the unreacted fatty alcohol content is less than 2%, preferably less than ½%.

### Detailed Description of the Invention

The fatty alcohols useful herein may be primary or secondary alcohols having straight or branched chains which can be either saturated or unsaturated, and may contain ether linkages. Preferably, the alcohols are primary saturated alcohols. Examples include dodecyl, tridecyl, tetradecyl, pentadecyl, hexadecyl, and octadecyl alcohols, and mixtures thereof. The preferred fatty alcohols are those containing from 12 to 14 carbon atoms.

The short chain alkylmonosaccharides, are e.g., the methyl, ethyl, propyl, butyl, and pentyl, preferably propyl or butyl, and most preferably butyl, fructosides or glucosides. The glucosides and fructosides are selected due to their availability and low cost, and the most preferred alkylmonosaccharide is derived from glucose. These compounds can be prepared separately, in a preliminary step, or as part of the first step since the corresponding short chain alcohols react with the corresponding saccharides much faster than the long chain fatty alcohols react.

The molar ratio of the long chain fatty alcohol to the short chain alkylmonosaccharide is between 1:4 and 4:1, preferably between 1:2 and 2:1, most preferably between 1:1 and 1.2:1. The level of long chain fatty alcohol is preferably kept as low as possible to facilitate the removal of unreacted fatty alcohol from the desired alkyl polysaccharide. An auxiliary solvent can be used to maintain fluidity. The lower levels of fatty alcohol also maximize the formation of the desired long chain alkylpolysaccharides especially at levels above 60%. The level of long chain alkylmonosaccharide in the finished product must be less than 60%, most preferably less than 50%.

The reaction is carried out at a temperature of from 90°C to 120°C, preferably above 100°C. Above 120°C there is excessive formation of colored materials and excessively fast saccharide chain growth. However, the reaction temperature should be as high as possible to minimize the time of the reaction.

If an auxiliary solvent is used, it should have a boiling point that will permit its easy removal for recycling. It should also be compatible with the short chain alcohol, the long chain alcohol, the saccharide and the alkyl saccharides and should not be reactive. Suitable auxiliary solvents include: toluene and C₈₋₁₂ hydrocarbons.

It is desirable that the resulting product contain a minimum of the short chain alkylsaccharides, which do not provide any substantial detergency benefits. However, care must be taken in removing the last amount of the short chain alcohol since this normally requires more stringent conditions and one wants to avoid removal of the long chain alcohol. Furthermore, if the reaction proceeds for too long a time, the average polysaccharide chain length becomes too long. Increasing the reaction time can be used to achieve longer polysaccharide chains since free saccharide reacts with the end of the saccharide chain preferentially as compared to the fatty alcohol. Accordingly, it is desirable to remove the short chain fatty alcohol rapidly and kill the catalyst by adding an alkaline material as soon as the desired product is achieved.

Known analytical techniques can be used to determine the structures of the alkylpolysaccharide surfactants herein; for example, to determine the saccharide chain length, the amount of butyl glucoside, the free fatty alcohol content, and the level of unreacted polysaccharide. More specifically, gas or liquid chromatography can be used to determine the unreacted alcohol content and the unreacted polysaccharide content respectively. Proton nmr can be used to determine the average saccharide chain length. The point of attachment of the hydrophilic portion of the molecule to the hydrophobic portion of the molecule can be determined by ¹³C nmr.

The alkylpolysaccharide surfactants are complex mixtures. Their components vary depending upon the nature of the starting materials and the reaction by which they are prepared. Analytical standards which are useful in calibrating instruments for analyzing the components of a particular alkylpolysaccharide surfactant can be obtained from Calbiochem Behring Co. LaJolla, California. These standards include those for octylglucoside (Calbiochem #494559), decylglucoside (Calbiochem #252715), and dodecylmaltoside (Calbiochem #3243555).

The combination of vacuum and temperature should not remove the long chain alcohol. Preferably the reaction takes place in a thin film, preferably at high Reynolds numbers (>720,000), as set forth hereinafter, to permit rapid removal of the short chain alcohol which results, and preferably the reaction takes place under a vacuum to assist in the rapid removal of the resulting short chain alcohol. Thin films can be achieved using, preferably, a wiped film evaporator or a drum evaporator, or mills in which two cylinders combine to form a thin film. In a mill it is desirable that one of the cylinders rotate faster than the other to impart a shearing and mixing action. the reaction mix is conveniently removed from a drum or cylinder by a doctor blade.

The length of the saccharide chain is primarily controlled by adjusting the ratio of the saccharide monomer to the fatty alcohol. For any given ratio there is a desired end point at which time the desired reaction is complete and beyond that point one effects undesired dehydration of the saccharide moieties.

The synthesis process can be monitored by following the percent of water contained in the distillate. (The distillate contains mainly lower fatty alcohol and long chain fatty alcohols.) The water level can be analyzed by collecting the distillate in fractions and titrating it with Karl Fischer reagent. The optimum end point is at the lowest water level in the distillate, preferably <0.1%. Further reaction increases the glycoside chain length rapidly and can make the product unsuitable for, e.g., detergent applications.

An in-line moisture monitor is ideal to control the process.

In one reaction in which the initial molar ratio of butyl glycoside to C₁₂₋₁₃ fatty alcohol was 3, the temperature was 118°C and the pressure varied from one cm. of Hg to one atmosphere, 1330 to (10⁵Pa), the percent water in the distillate varied with the amount of distillate collected as follows:

| | | | | | | | | |
|---|---|---|---|---|---|---|---|---|
| Distillate (ml) | 50 | 140 | 270 | 320 | 360 | 375 | 380 | 396 |
| %H₂O | 12.4 | 4.1 | 0.9 | 0.2 | 0.08 | 0.25 | 0.3 | 0.15 |

The desired end point occurs when the percent of water in the distillate is less than 0.2, preferably less than 0.1, most preferably less than 0.08.

The acid catalysts can be any of the conventional acids such as sulfuric acid, hydrochloric acid, phosphoric acid, phosphorous acid and toluene sulfonic acid, and other Lewis acid catalysts. The amount of acid catalyst used is between 0.0001 mole per mole of saccharide monomer and 0.02 mole per mole of saccharide monomer, preferably between 0.005 and 0.01, most preferably between 0.001 and 0.006. The amount of catalyst used can control the speed of reaction. When larger amounts of catalysts are used the neutralized catalyst should be one that is compatible with the end use of the surfactant. Sulfuric acid is the preferred catalyst.

### Removal of the Fatty Alcohol

The fatty alcohol can be removed from the product of the first step by distillation or by a solvent extraction technique. The fatty alcohol and any auxiliary solvent are removed after the catalyst has been destroyed by neutralization. The preferred method of fatty alcohol removal is to form a thin film of the reaction product containing the neutralized catalyst and apply heat and a vacuum. A wiped film evaporator is a particularly preferred piece of equipment for removing the fatty alcohol.

It has now been found that the use of a thin film evaporator and a vacuum permits the removal of the fatty alcohols and/or solvent of this invention to a level below 2% and even below 1/2% without any appreciable change in the color of the product. This discovery is especially important when one is preparing alkylpolysaccharides from alcohols containing more than 10 carbon atoms where the boiling point of the fatty alcohol, even under a high vacuum, is very high and prolonged exposure to high temperature leads to decomposition of the polysaccharide. With alkyl polysaccharides in which the alkyl group contains more than 10 carbon atoms, it is preferred to have a small amount of short chain alkyl (C₁₋₅) saccharide present to maintain the fluidity thus allowing lower temperatures to be used.

In the removal process of this invention the product is formed into a thin film at a temperature of from 160°C to 200°C, preferably from 160°C to 180°C, most preferably from 160°C to 170°C and under a vacuum of from 0.1 mm Hg to 20 mm Hg. (13.3 to 2660 Pa) preferably from 0.1 mm Hg to 5 mm Hg, (13.3 to 666 Pa) most preferably from 0.1 mm Hg to 3 mm Hg (13.3 to 400 Pa). The thin film during the reaction and the removal steps is formed in a thin film evaporator which gives a film with a thickness of from 1 mm to 10 mm and a Reynolds number of at least 20,000, preferably at least 50,000, and more preferably 100,000. The film in such evaporator is preferably less than 5 mm at its thinnest and more preferably less than 5 mm in the wave.

### Preferred Product

The process defined hereinbefore can be used to prepare a preferred alkylpolysaccharide detergent surfactant having superior properties with respect to detergency and suds boosting for other detergent surfactants. This preferred alkylpolysaccharide has the formula RO(Z)ₓ is which R is an alkyl group containing from 12 to 18 carbon atoms; Z represents glucoside or fructoside moiety; x averages from 1.6 to 2-3/4; the amount of alkylpolysaccharide in which x is greater than 6 is less than 10%, preferably less than 5%; the amount of the alkylpolysaccharide in which x is 1 is less than 60%, preferably less than 50%, and the defined material is associated with no more than 10% alkylsaccharides and polysaccharides wherein the alkyl group contains 1 to 5 carbon atoms and with no more than 2% alcohol containing an R group.

Preferably in the above compound R is a straight chain saturated alkyl group and preferably the R groups contain from 12 to 14 carbon atoms. An even more preferred average of x is from 1.7 to 2.5. The preferred Z group is a glucoside group.

The above preferred alkylpolysaccharide preferably constitutes at least 90% of the alkylpolysaccharide material present. It is difficult to achieve an alkylpolysaccharide having the appropriate alkyl chain length and average x without either exceeding the desired amount of fatty alcohol or monosaccharide on the one hand, or providing excess material in which x exceeds 6 on the other hand. It is also difficult to maintain a good color in alkyl polysaccharide detergent surfactants to permit their incorporation into detergent compositions, and to minimize the amount of short chain alkylpolysaccharide present.

The process described hereinbefore achieves the desired product by minimizing the temperature to which the material is exposed, especially while an acid catalyst is present; maximizing the speed at which the short chain alcohol is removed; killing the catalyst as soon as the desired end point has been reached; and then removing the fatty alcohol, preferably by a process which minimizes the time and the temperature to which the desired product is exposed.

Surprisingly, it has been discovered that the increase in the alkyl chain length from 10 to 12 results in a very large decrease in the reactivity of the fatty alcohol. It also increases considerably the difficulty involved in removing the fatty alcohol without exceeding the decomposition temperature of the polysaccharide chain. The combination process disclosed herein achieves the desired product.

The importance of the limits on the preferred alkylpolysaccharide detergent surfactant are documented hereinafter in the examples. In order to obtain maximum performance, x needs to be as low as possible while maintaining water solubility. The desired products have an HLB of from 7 to 30, preferably from 10 to 20, and a critical micelle concentration of from 10 ppm to 1000 ppm, preferably from 20 ppm to 500 ppm.

### Example I

50 ml of n-butanol, 10 g anhydrous glucose, 20 ml n-dodecanol and 0.0534 g of p-toluene sulfonic acid were added to a 100 ml 3-neck flask with stirring. The reaction mixture was refluxed at 115%-117°C for 2 hours. The n-butanol was then removed as fast as possible with the help of partial vacuum while keeping the temperature at 100°C. to 120°C. The reaction was kept at 120°C, 5 cm Hg vacuum (6660 Pa) for 40 minutes. 0.027 g of Na₂CO₃ was used to neutralize the reaction mixture. The unreacted n-dodecanol was then distilled off using a Wiped Film Evaporator by Pope at a temperature of 165°C and a vacuum of 2 mm Hg (266 Pa). The final sample is a crispy solid with the following analyses: (In the following, e.g. dodecanol polyglycoside with n glucose moieties is abbreviated C₁₂Gₙ). Average glucose number per alkyl chain; 2.0, weight percent of n-butyl oligoglycoside: <5.9%, C₁₂, G₁ : ~40%, C₁₂G₂: 21%, C₁₂G₃ 13%, C₁₂G₄ 9.8%, C₁₂G₅ 8%, >C₁₂G₆, <10%, C₁₂-OH: 0.46%.

### Example II

500 ml n-butanol, 48 g anhydrous glucose, 200 ml Neodol 23 and 0.0534 g of p-toluene sulfonic acid were reacted. The reaction was carried out in a 1000 ml flask with vigorous stirring. After n-butanol removal, reaction condition was set at 118°C, 2 cmHg (2660 Pa) vacuum until 103% of the added n-butanol volume was collected. (Water) After neutralizing in the usual manner, the sample was dried, as in Example I, through the wiped film evaporator. The product was analyzed to be : average glycoside number per alkyl chain: 2.0, weight percent of n-butyl aligoglycoside <1%, C_{12,13}G₁ ~ 49%, C_{12,13}G₂ : 19%, C_{12,13}G₃: 11,5%, C_{12,13}G₄: 8,4%, C_{12,13}G₅: 4,44%, C_{12,13}G₆: 3.85%.

### Example III

500 ml n-butanol, 192 g anhydrous dextrose, 200 ml Neodol 23 and 0.0534 g p-toluene sulfonic acid. The reaction was carried out in the same manner as Example II. The final product was analyzed to be: average glucose number per alkyl chain: <2.5, C_{12,13}G₁: 35%, C_{12,13}G₂: 20%., C_{12,13}G₃: 15%, C_{12,13}G₄: 12.6%, C_{12,13}G₅: 9.8%, C_{12,13}G₆: 7.6%.

### Comparative Example IV

480 g anhydrous dextrose, 6.0 g p-toluene sulfonic acid, 960 g Neodol-23 (C_{12 - 13} fatty alcohol) and 3600 ml n-butanol were refluxed at 117°C for 1 hour. The n-butanol was distilled off under atmospheric pressure for 45 minutes at 120°C. The pressure was reduced gradually to 50 mm Hg (6660 Pa) and the temperature was maintained at 130°C for 1.5 hours. The reaction mixture was neutralized with 3 g sodium carbonate in 20 ml H₂O. The excess alcohol was washed away with acetone. The product had the following analyses: 27% butyl polyglycosides, 10.2 glucose units/per molecule. This indicated the temperature was too high and too much catalyst was used. This favored the polysaccharide chain length polymerization without removing the butyl polyglycosides.

### Example V

960 g anhydrous dextrose, 2000 ml Neodol-23. 5000 ml n-butanol and 1.2 g concentrated sulfuric acid were refluxed at 117°C for 2 hours. n-butanol was then removed at 117°C, 2 cm Hg (2660 Pa) pressure. The reaction mixture was neutralized with Na₂CO₃ solution. The excess Neodol-23 was then removed by a 51 cm (2 inch) Pope Wiped Film Evaporator operated at 160-170°C, 2mm Hg (266 Pa) pressure. The solution residence time in the hot zone was approximately 1 minute. The resulting product had a light brown color. Melting point about 130°C. It was cooled to room temperature and was easily ground into a yellowish powder. Analyses showed a content of 0.45% Neodol-23. It dissolved readily in water and gave a yellowish clear solution.

## Claims

1. The process of making purified alkyl polysaccharide by removing alcohol from a mixture with an alkyl polysaccharide by heating the mixture under vacuum in a thin film evaporator wherein the saccharide is glucoside or fructoside, characterised in that the alkyl group contains 12 to 18 carbons and the alcohol is the corresponding fatty alcohol, the purified alkyl polysaccharide has an average chain length of 1½ to 3 and includes less than 10% of C₁₋₅ alkyl saccharide and polysaccharide and less than 10% of alkyl polysaccharide having a chain length of 6 or more and has an alkyl monosaccharide content of less than 60% and a fatty alcohol content of less than 2%, the thin film evaporator provides, in operation, a Reynolds number of at least 20,000 and a film thickness of less than 10mm, and a temperature of from 160°C to 200°C and a vacuum of from 0.1 to 20mm of mercury (13.3 to 2660Pa), and the mixture is neutralised before the removal of fatty alcohol.

2. The process of claim 1 wherein the Reynolds number is at least 50,000, the temperature is from 160°C to 180°C, and the vacuum is from 0.1 to 5mm of mercury (13.3 to 660Pa).

3. The process of claim 1 or claim 2 wherein the fatty alcohol is removed to a level of less than ½%.

## Patentansprüche

1. Verfahren zur Herstellung von gereinigtem Alkylpolysaccharid durch Entfernen von Alkohol aus einer Mischung mit einem Alkylpolysaccharid durch Erhitzen der Mischung unter Vakuum in einem Dünnschichtverdampfer, wobei das Saccharid Glucosid oder Fructosid ist, dadurch gekennzeichnet, daß die Alkylgruppe 12-18 Kohlenstoffatome enthält und der Alkohol der korrespondierende Fettalkohol ist, das gereinigte Alkylpolysaccharid eine durchschnittliche Kettenlänge von 1 1/2 bis 3 aufweist und weniger als 10% an C₁₋₅-Alkylsaccharid und Polysaccharid und weniger als 10% an Alkylpolysaccharid mit einer Kettenlänge von 6 oder mehr beinhaltet und einen Alkylmonosaccharidgehalt von weniger als 60% und einen Fettalkoholgehalt von weniger als 2% aufweist, wobei der Dünnschichtverdampfer während des Betriebs eine Reynolds-Zahl von mindestens 20.000 und eine Filmdicke von weniger als 10 mm, und eine Temperatur von 160°C bis 200°C und ein Vakuum von 0,1 bis 20 mm Quecksilber (13,3 bis 2660 Pa) vorsieht, und die Mischung vor der Entfernung von Fettalkohol neutralisiert wird.

2. Verfahren nach Anspruch 1, wobei die Reynolds-Zahl mindestens 50.000, die Temperatur 160 bis 180°C, und das Vakuum 0,1 bis 5 mm Quecksilber (13,3 bis 660 Pa) betragen.

3. Verfahren nach Anspruch 1 oder 2, wobei der Fettalkohol bis zu einem Anteil von weniger als 1/2 % entfernt wird.

## Revendications

1. Procédé de préparation d'alkylpolysaccharide purifié par élimination d'alcool à partir d'un mélange avec un alkylpolysaccharide en chauffant le mélange sous vide dans un évaporateur à film mince où le saccharide est un glucoside ou un fructoside, caractérisé en ce que le groupe alkyle contient 12 à 18 atomes de carbone et que l'alcool est l'alcool gras correspondant, l'alkylpolysaccharide purifié a une longueur de chaîne moyenne de 1 1/2 à 3 et renferme moins de 10 % d'alkyl(en C₁₋₅) saccharide et polysaccharide et moins de 10% d'alkylpolysaccharide ayant une longueur de chaîne de 6 ou plus, et a une teneur en alkylmonosaccharide inférieure à 60 % et une teneur en alcool gras inférieure à 2 %, l'évaporateur à film mince fournit, en fonctionnement, un nombre de Reynolds d'au mois 20 000 et une épaisseur de film inférieure à 10 mm, et une température de 160°C à 200°C et un vide de 0,1 à 20 mm de mercure (13,3 à 2 660 Pa), et le mélange est neutralisé avant l'élimination de l'alcool gras.

2. Procédé selon la revendication 1, dans lequel le nombre de Reynolds est d'au moins 50 000, la température est de 160°C à 180°C, et le vide est de 0,1 à 5 mm de mercure (13,3 à 660 Pa).

3. Procédé selon la revendication 1 ou 2, dans lequel l'alcool gras est éliminé jusqu'à une teneur inférieure à 1/2 %.
